# NEUE EUROPÄISCHE PATENTSCHRIFT

(11) **EP 1 935 446 B2**
(45) Veröffentlichungstag und Bekanntmachung des Hinweises auf die Entscheidung über den Einspruch: **04.05.2022**
(45) Hinweis auf die Patenterteilung: 28.12.2011
(21) Anmeldenummer: 07400032.4
(22) Anmeldetag: 18.12.2007
(51) Int. Cl.: A61M 16/00, A61M 16/12, G09B 23/28, A61H 31/00

(54) **Vorrichtung und Verfahren zur Abgabe von Sauerstoff im Rahmen einer Reanimation**
Device and method for releasing oxygen during a reanimation
Dispositif et procédé d'administration d'oxygène dans le cadre d'une réanimation

(30) Priorität: 20.12.2006 DE 102006060981
(43) Veröffentlichungstag der Anmeldung: 25.06.2008
(73) Patentinhaber: WEINMANN Emergency Medical Technology GmbH + Co. KG, 22525 Hamburg (DE)
(72) Erfinder: Palm, Ulrich, 22848 Norderstedt (DE)
(74) Vertreter: Klickow & Wetzel PartGmbB

(56) Entgegenhaltungen:
- EP-A- 1 369 144
- WO-A-01/15761
- GB-A- 934 973
- US-A- 3 229 689
- US-A- 4 520 811
- US-A1- 2006 111 749

## Beschreibung

Die Erfindung betrifft eine Vorrichtung zur Vergabe von Zeittakten für einen Helfer bestehend aus einer Steuereinrichtung, die an mindestens einen spannungsgenerator angeschlossen ist, welcher mit mindestens einer Elektrode verbindbar ist, und die mit einer sauerstoffleitenden Verbindung mit einer Sauerstoffquelle, einem Sauerstoffventil und einem Sauerstoffausgang sowie mindestens einem Signalgeber versehen ist, der zur Ausgabe akustischer und/oder visueller Signale ausgebildet ist.

An öffentlichen Orten, Büros sowie in Wohnungen finden vermehrt Defibrillatoren Einzug. Diese können einem Patienten, bei dem das Herz keine Aktivität mehr aufweist oder fibrilliert, also unkontrollierte Muskelzuckungen aufweist, in Zusammenarbeit mit Laienhelfern und/ oder Professionals schnelle Hilfe bieten.

Diese Defibrillatoren verfügen über Spannungsgeneratoren, welche einen Elektroschock über am Patienten angelegte Elektroden abgeben können, um für eine gewisse Zeit die biologischen unkontrollierten Potentiale bzw. ungerichtete Reizleitungen im Herzen gleichzeitig auszurichten und die Möglichkeit einer optimalen Reizweiterleitung durch gerichtete Reizleitungen wiederherzustellen. Daneben ist es besonders vorteilhaft für derartige Patienten, gleich zu Beginn Sauerstoff zu erhalten, da die meisten Patienten zusätzlich zu ihrem fibrillierenden Herzen einen Atemstillstand aufweisen und es in diesem Zuge zu einer Sauerstoffunterversorgung des Gehirns mit extremen Schädigungen des Hirn-sowie Herzmuskelgewebes kommen kann.

Dieser Unterversorgung wird durch Sauerstoffflaschen mit Druckminderer und einem einstellbarem Flow entgegengewirkt bzw. diese werden direkt an einen Beatmungsbeutel angeschlossen, den ein Laienhelfer in den meisten Fällen aber nicht zur Hand hat.

Sauerstoffquellen in Defribrillatoren sind beispielsweise in der Schrift EP 1369144 beschrieben.

Um auch ohne Beatmungsbeutel und für fast alle Laienhelfer eine Möglichkeit zu bieten, den Patenten zu beatmen, kann eine Beatmungsmaske verwendet werden. Dazu legt der Helfer die Maske dem Patienten an, positioniert den Maskenwulst um die Mund- und Nasenöffnungen des Patienten und beatmet diesen mit Hilfe seiner Exspirationsluft über die Maske. Dies sorgt in den meisten Fällen für eine ausreichende oxygenierung des Patienten. Ferner ist durch die Maske gesichert, dass dem Helfer ausreichend Schutz vor eventuellen Infektionen durch den Patienten gegeben wird.

Viele Masken besitzen Rückschlagventile, welche die exspiratorische Patientenluft nicht zum Helfer hin, sondern direkt zur Umgebung ausströmen lassen. Auch bei den Masken gibt es die Möglichkeit, über einen Anschlussstutzen und eine angeschlossene Gasquelle, Sauerstoff in den Maskeninnenraum einzuleiten und bei der Inspiration (Beatmung durch den Helfer) den Sauerstoff in die Lunge des Patienten zu transportieren.

Im Falle einer Reanimation ist es zudem vorteilhaft, die Ventilation des Kreislaufsystems durch Herzdruckmassagen auf den Thorax aufrecht zu erhalten. Dies sollte mit einer gewissen Frequenz geschehen. Dazu ist in einigen Defibrillatoren bereits ein Metronom eingebaut.

Da der Sauerstoff bei den bisher bekannten Vorrichtungen meist im Dauerflow aus der Sauerstoffquelle ausströmt, wird dieser auch in den Phasen, in denen der Patient nicht beatmet wird, der Maske oder einem Beatmungsbeutel zugeführt und somit nicht bzw. ineffizient genutzt.

Daher ist das Volumen des Druckgasbehältnisses in den meisten Fällen sehr groß gewählt, um den nötigen Sauerstoff auch über die benötigte Zeit zur adäquaten Versorgung des Patienten applizieren zu können.

Das Medium Sauerstoff steht, wenn dieser nicht aus einem hauseigenen System entnommen wird, somit nur in begrenztem Umfang zur Verfügung.

Dieser Umstand lässt die Behälter sehr groß und die Anwendung unpraktikabel werden, da ein schweres und großes Gerät in einer Not- und Paniksituation verwendet werden muss.

Ferner bietet die Kombination weiterer Ideen einen deutlichen Mehrwert bei der Zuführung von Sauerstoff in einer Reanimationssituation.

Aus der US 2006/111,749 A1 ist bereits eine Vorrichtung zur Vergabe von Zeittakten für einen Helfer bekannt, die eine Steuereinrichtung und einen Spannungsgenerator aufweist. Der Spannungsgenerator ist mit einer Elektrode verbindbar. Eine Sauerstoff leitende Verbindung mit einer Sauerstoffquelle ist von einem Sauerstoffventil steuerbar. Ein verwendeter Signalgeber ist zur Ausgabe von akustischen oder visuellen Signalen ausgebildet.

Die WO 01/157661 A beschreibt die Verwendung eines Beatmungsbeutels zur manuellen Beatmung eines Patienten.

In der GB 934973 A wird ebenfalls eine Beatmungsvorrichtung für einen Patienten beschrieben. Die US-A-4,520,811 beschreibt eine weitere manuell steuerbare Beatmungsvorrichtung, an die eine Druckgasflasche mit Sauerstoff anschließbar ist.

Aus der US-A-3,229,689 ist eine spezielle Beatmungsvorrichtung bekannt, die es einem Helfer ermöglicht, unter Zumischen von Sauerstoff aus einer Druckgasflasche einen Patienten zu beatmen.

Aufgabe der vorliegenden Erfindung ist es, eine Vorrichtung der einleitend genannten Art derart zu verbessern, daß eine Verminderung des Verbrauches an Sauerstoff unterstützt wird.

Diese Aufgabe wird erfindungsgemäß durch die Merkmale von Patentanspruch 1 gelöst.

Weitere Aufgabe der vorliegenden Erfindung ist es, ein Verfahren der einleitend genannten Art derart zu verbessern, daß eine Verringerung des Verbrauches an Sauerstoff unterstützt wird.

Diese Aufgabe wird erfindungsgemäß durch die Merkmale von Patentanspruch 14 gelöst.

Mit der vorliegenden Erfindung wird erreicht, dass nur noch in den Momenten, in denen der Patient auch wirklich den Sauerstoff aufnehmen kann, also in den inspiratorischen Beatmungsphasen, die Steuerung eines Defibrillators eine erhöhte Abgabe an Sauerstoff freisetzt.

Erstmals wird die Kombination von einer geringen Größe eines Defibrillators mit einer optimierten Sauerstoffabgabe aus einem kleinen Gasdruckbehälter innerhalb des Defibrillators mit der Vorgabe einer optimalen Beatmungs- und HDM-Frequenz bereitgestellt. Die Vorrichtung gibt dem Helfer den Takt mittels Metronom vor, in welchem er den Kreislauf einer leblosen Person durch HDM (Herzdruckmassage) aufrecht erhalten kann. Nach einer fest vorgegebenen Anzahl an HDM folgt eine fest vorgegebene Anzahl an Beatmungen durch den Helfer, bei denen zeitgleich die Sauerstoffabgabe erfolgt. Durch die Steuerung des Defibrillators wird der Sauerstoff von einem Gasdruckbehältnis oder einem Hausdrucksystem an einen Ausgang befördert. An diesen kann wahlweise eine weitere Vorrichtung zur Beatmung eines Patienten angeschlossen sein.

In einer Ausführungsform der Erfindung verfügt ein Defibrillator über eine Sauerstoffeinheit und einen akustischen und/oder visuellen/optischen Signalgeber zur Ausgabe von akustischen und / oder optischen Signalen. Dieser Signalgeber gibt dem Helfer getaktete Signale für eine optimale Frequenz zur HDM vor, die in den meisten Fällen eines Herzstillstandes bzw. fibrillierenden Herzens nötig ist. Nach einer fest vorgegebenen Anzahl an ersten Signalen, vorzugsweise nach 30 Signalen, wird dem Helfer eine fest vorgegebene Anzahl an zweiten Signalen gegeben, den Patienten mit einer fest vorgegeben Anzahl an Atemzügen zu ventilieren (Beatmungsphase), vorzugsweise 2 Atemzüge. Die ersten Signale geben die Frequenz der HDM, die zweiten Signale die Frequenz der Beatmungsphase vor. Beide Frequenzen können gleich sein oder sich bedingt durch den Wechsel von Handgriffen unterscheiden.

Des Weiteren kann eine Totzeitphase dazwischen liegen, die den Handgriffwechsel, welcher der Helfer von der Beatmung zur HDM und umgekehrt vollführen muss, berücksichtigt. Die Reihenfolge des ersten und zweiten Signals kann variieren, um beispielsweise gleich am Anfang der Inbetriebnahme des Gerätes für eine Sauerstoffabgabe zu sorgen.

Die ersten und zweiten Signale sind vorzugsweise unterschiedlich, um dem Helfer die verschiedenen Aufgaben zur optimalen Reanimation des Patienten zu verdeutlichen. Beispielsweise können bei den visuellen Signalen unterschiedliche Farben zum Einsatz kommen, sowie bei den liche Farben zum Einsatz kommen, sowie bei den akustischen Signalen andere Töne.

Die Signale können akustisch über einen Lautsprecher an die Aussenwelt abgegeben werden. In den durch das Gerät vorgegeben Beatmungsphasen wird durch die Steuerung die Sauerstoffgabe freigeschaltet. Dies kann über ein Ventil erfolgen. Bei einer Freischaltung gibt der Sauerstoffdruckbehälter einen erhöhten Flow größer 2000ml/min ab, vorzugsweise 8000ml/min.

Diese Sauerstoffmenge wird mit dem vom Helfer ausgehenden Beatmungsflow dem Patienten appliziert und sorgt für eine optimale oxygenierung des Patienten.

In einer weiteren Ausführungsform der Erfindung besteht die Vorrichtung aus mindestens einem Defibrillator, einem Signalgeber und einer Sauerstoffeinheit, bei der die Steuerung über einen Sensor die Beatmungsbemühungen des Ersthelfers/Professionals detektiert und das Ventil zur Sauerstoffabgabe öffnet.

Dies kann in einer weiteren Ausführungsform mit einer fest vorgegebenen oder einer einstellbaren Öffnungszeit geschehen. So wird auch nur dann Sauerstoff freigesetzt, wenn dieser auch durch den Beatmungsflow vom Helfer und/oder vom Beatmungsbeutel in die Atemwege des Patienten gelangen kann.

In der Maske befindet sich für gewöhnlich ein gewisser Totraum, welcher sich mit Umgebungsluft füllt bzw. die Exspirationsluft des Patienten enthält. Dieses Luftvolumen gelangt bei der nächsten Inspiration wieder in die Atemwege, insbesondere in die Alveolen, des Patienten, da das Luftvolumen diese zuerst erreicht. Da jedoch die Alveolen die bevorzugte Austauschregion von CO2 und 02 sind, ist in einer weiteren Ausführungsform der Erfindung daran gedacht, vor dem Beatmen des Patienten bzw. in den Phasen der HDM, das Sauerstoffventil mit einem geringen Flow zu öffnen, so dass sich der Totraum der Maske und der oberen Atemwege bereits mit Sauerstoff anreichern kann. Das überschüssige Sauerstoff-Luft-Gemisch kann über das Ventil entweichen.

In den Zeichnungen sind Ausführungsbeispiele der Erfindung schematisch dargestellt. Es zeigen:
- Fig. 1: Ein Längsschnitt durch ein Beatmungsventil, das mit einem Schnabelventil ausgestattet ist,
- Fig. 2: die Anordnung gemäß Fig. 1 in einer anderen Positionierung des Schnabelventils und
- Fig. 3: ein Diagramm zur Veranschaulichung der zeitlichen Zuordnung von Phasen für eine Herzdruckmassage und eine Beatmung.

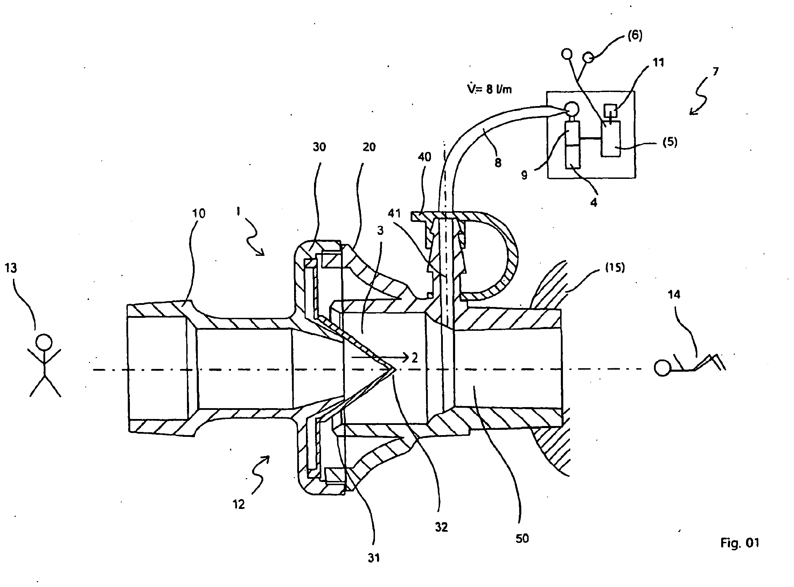

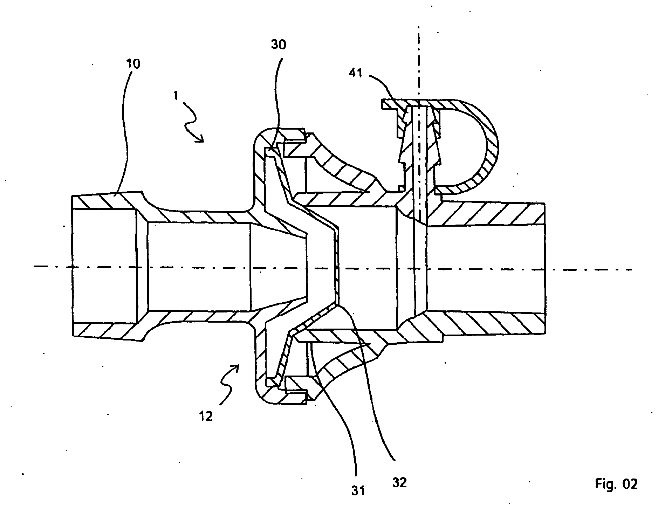

In Fig. 1 ist ein Beatmungsventil (1) dargestellt. Dies verfügt über ein Mundstück (10) für den Helfer (13) und ein Einwegeventil (12) bestehend aus einer Membran (30), einem Ventilsitz (31) und einem Schnabelventil (32). Bei der Beatmung folgt die helferseitige Exspirationsluft dem dargestellten Pfeil (2). Durch den vom Helfer aufgebrachten Druck schließt sich das Einwegeventil (12) entsprechend Fig. 2 durch Aufpressen der Membran (30) auf den Dichtsitz (31). Die Luft des Helfers mischt sich mit dem aus dem seitlichen Eingangsstutzen (41) strömenden Sauerstoff und gelangt über ein hier als Maske dargestelltes Patienteninterface in die Alveolen des Patienten (14).

An diesen Eingangsstutzen (41) ist die erfindungsgemäße Vorrichtung angeschlossen, die einen Sauerstoffdruckbehälter (4) aufweist, welcher über ein Sauerstoffventil (9), eine Steuerung (5), zwei Elektroden (6) zum Anbringen an den Patienten, mindestens einen Spannungsgenerator (11) und eine Sauerstoffverbindungsleitung (8) zum Anschluss an das Beatmungsventil (1) verfügt.

Der Totraum (50) kann in einer Ausführungsform der Erfindung mit Sauerstoff über die Verbindungsleitung (8) angereichert werden.

An den patientenseitigen Anschluss kann eine Beatmungsmaske (15) angeschlossen werden, welche auf das Gesicht des Patienten gedrückt wird.

Das in Fig. 1 und 2 dargestellte Schnabelventil (32) besteht typischerweise aus Segmenten, die bei einer helferseitigen Druckbeaufschlagung auseinandergespreizt werden und einen Durchgang vom Mundstück (10) in Richtung auf den Totraum (50) bereitstellen. Aufgrund der elastischen Eigenschaften der Membran (30) bzw. des Schnabelventils (32) kehrt dieses bei einer Beendigung der helferseitigen Druckaufschlagung von dem in Fig. 2 dargestellten Betriebszustand in die in Fig. 1 dargestellte Grundpositionierung zurück. In dieser Positionierung ist das Schnabelventil (32) geschlossen und eine Rückatmung in Richtung auf den Helfer wird vermieden.

Ohne Anbringung der Verbindungsleitung (8) ist es möglich, den Eingangsstutzen (41) unter Verwendung einer Verschlußkappe (40) abzudecken und somit gegenüber von Verunreinigungen zu schützen. Die Verschlußkappe (40) verhindert ebenfalls ein seitliches Austreten von Beatmungsluft für den Fall, daß keine Zumischung von Sauerstoff vorgesehen ist.

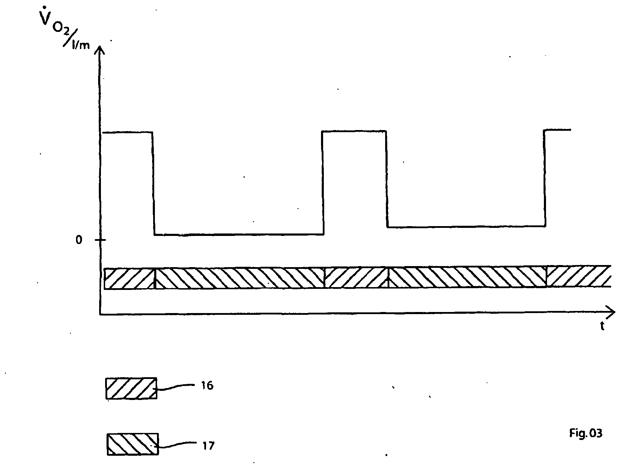

Fig. 3 veranschaulicht eine optimierte Zuordnung von Phasen für die Durchführung einer Herzdruckmassage (17), sowie Phasen für die Durchführung einer Beatmung (16) sowie der entsprechenden Phasen für die Sauerstoffdosierung.

Wenn man die Frequenzen fest vorgibt, kann es sein, daß der Helfer überfordert ist, da er nicht so schnell hinterherkommt. Deshalb sollte das Gerät erkennen, wann der Helfer beatmet bzw. die Herzdruckmassage (über Impedanzinfo) fortführt. Der Takt setzt dann immer bei der ersten Aktion ein und wird für 2 und 15 Takte weitergeführt.

## Patentansprüche

1. Vorrichtung zur Vergabe von Zeittakten für einen Helfer, bestehend aus einer Steuereinrichtung, die an mindestens einen Spannungsgenerator angeschlossen ist, welcher mit mindestens einer Elektrode verbindbar ist, und die mit einer sauerstoffleitenden Verbindung mit einer Sauerstoffquelle, einem Sauerstoffventil und einem Sauerstoffausgang sowie mindestens einem Signalgeber versehen ist, der zur Ausgabe akustischer und/oder visueller Signale ausgebildet ist, **dadurch gekennzeichnet, dass** die Vorrichtung als Teil eines Defibrillators ausgebildet ist, dass die Elektrode zur Durchführung einer Defibrillation ausgebildet ist, dass der Spannungsgenerator zur Erzeugung einer für die Durchführung einer Defibrillation geeigneten Spannung ausgebildet ist und dass der Signalgeber dem Helfer getaktete Signale für eine optimale Frequenz zur Herzdruckmassage vorgibt, wobei nach einer fest vorgegebenen Anzahl an ersten Signalen, dem Helfer eine fest vorgegebene Anzahl an zweiten Signalen vorgegeben wird, den Patienten mit einer fest vorgegeben Anzahl an Atemzügen zu ventilieren (Beatmungsphase) und wobei in den durch das Gerät vorgegebenen Beatmungsphasen durch die Steuerung die Sauerstoffgabe freigeschaltet wird.

2. Vorrichtung nach Anspruch 1, **dadurch gekennzeichnet, dass** der akustische Signalgeber ein Lautsprecher ist.

3. Vorrichtung nach Anspruch 1, **dadurch gekennzeichnet, dass** der visuelle Signalgeber eine farbliche LED, ein Display und/oder eine sonstige Licht emittierende Strahlenquelle ist.

4. Vorrichtung nach Anspruch 1, **dadurch gekennzeichnet, dass** an den Sauerstoffausgang über eine Verbindungsleitung (8) eine Beatmungsventil (1) angeschlossen ist.

5. Vorrichtung nach Anspruch 4, **dadurch gekennzeichnet, dass** das Beatmungsventil (1) über ein Innenvolumen verfügt, in welches Sauerstoff entlastbar ist.

6. Vorrichtung nach einem der vorhergehenden Ansprüche, **dadurch gekennzeichnet, dass** mindestens ein Sensor mit der Steuereinheit verbunden ist, welcher die Beatembemühungen des Helfers detektiert.

7. Vorrichtung nach einem der vorhergehenden Ansprüche, **dadurch gekennzeichnet, dass** mindestens ein Sensor mit der Steuereinheit verbunden ist, welcher die Herzdruckmassagen des Helfers detektiert.

8. Vorrichtung nach den beiden obigen Ansprüchen, **dadurch gekennzeichnet, dass** bei Detektion einer Beatmungsbemühung der Taktgeber für die Beatmung einsetzt und das Sauerstoffventil offen ist und/oder bei Detektion einer Druckbemühung der Taktgeber für die Herz-Druckmassage einsetzt und/oder das Sauerstoffventil geschlossen ist.

9. Vorrichtung nach einem der Ansprüche 1 bis 8, **dadurch gekennzeichnet, dass** im Bereich des Beatmungsventils (1) ein Einwegeventil (12) angeordnet ist.

10. Vorrichtung nach Anspruch 9, **dadurch gekennzeichnet, dass** das Einwegeventil (12) eine Membran (30) aufweist.

11. Vorrichtung nach Anspruch 9 oder 10, **dadurch gekennzeichnet, dass** das Einwegeventil (12) ein Schnabelventil (32) aufweist.

12. Vorrichtung nach einem der Ansprüche 1 bis 11, **dadurch gekennzeichnet, dass** das Einwegeventil (12) in einer Grundposition eine Verbindung zwischen einem Innenraum einer Beatmungsmaske und einer Umgebung freigibt.

13. Vorrichtung nach einem der Ansprüche 1 bis 12, **dadurch gekennzeichnet, dass** das Einwegeventil (2) in einem Betriebszustand bei einer Druckbeaufschlagung durch einen Helfer den Verbindungsweg zwischen der Atemmaske und der Umgebung verschließt und einen Durchgang von einem Mundstück (10) zum Innenraum der Beatmungsmaske freigibt.

14. Verfahren zur Vergabe von Zeittakten für einen Helfer unter Verwendung der Vorrichtung nach Patentanspruch 1, bestehend aus mindestens zwei Phasen, wobei die erste Phase, Herzdruckmassage-Phase, und die zweite Phase, Beatmungsphase, verschiedene Zeitdauern besitzen können, wobei die Phasen jeweils durch fest vorgebbare Frequenzen an akustischen und/oder visuellen Signalen gekennzeichnet sind und bei dem Sauerstoff von der Sauerstoffquelle über das Sauerstoffventil an den Sauerstoffausgang abgegeben werden kann, **dadurch gekennzeichnet, dass** das Verfahren vom Defibrillator durchgeführt wird und dass in mindestens einer Phase eine Sauerstoffabgabe höher als in den übrigen Phasen ist und dass eine Steuerung des Defibrillators eine Sauerstoffabgabe in Beatmungsphasen freischaltet.

15. Verfahren nach einem der vorhergehenden Ansprüche, **dadurch gekennzeichnet, dass** die erhöhte Sauerstoffabgabe nur in der Beatmungsphase erfolgt.

16. Verfahren nach einem der vorhergehenden Ansprüche, **dadurch gekennzeichnet, dass** in der ersten Phase ein geringer Sauerstoffflow aus dem Ausgang der Sauerstoffquelle entweicht.

17. Verfahren nach einem der vorhergehenden Ansprüche, **dadurch gekennzeichnet, dass** ein Sensor Beatmungsbemühungen detektiert und nach einer entsprechenden Detektion die erhöhte Sauerstoffabgabe eingeleitet wird.

18. Verfahren nach einem der vorhergehenden Ansprüche, **dadurch gekennzeichnet, dass** die erhöhte Sauerstoffabgabe für einen festen Zeitraum abgegeben wird.

19. Verfahren nach einem der vorhergehenden Ansprüche, **dadurch gekennzeichnet, dass** mindestens ein Sensor mit der Steuereinheit verbunden wird, welcher Herzdruckmassagen des Helfers detektiert.

20. Verfahren nach Anspruch 19, **dadurch gekennzeichnet, dass** bei Detektion einer Beamtungsbemühung der Taktgeber für die Beatmung einsetzt und das Sauerstoffventil offen ist und/oder bei Detektion einer Druckbemühung der Taktgeber für die Herz-Druckmassage einsetzt und/oder das Sauerstoffventil geschlossen wird.

## Claims

1. Device for issuing clock pulses for a helper, consisting of a controller, which is connected to at least one voltage generator, which can be connected to at least one electrode, said controller being provided with an oxygen-carrying connection to an oxygen source, an oxygen valve and an oxygen outlet and at least one signal generator, which is in a form for outputting acoustic and/or visual signals, **characterized in that** the device is designed as part of a defibrillator, that the electrode is designed to perform a defibrillation, that the voltage generator is designed to generate a voltage suitable for performing a defibrillation, and that the signal generator gives to the helper clocked signals for an optimal frequency for cardiac massage, wherein after a fixed predetermined number of first signals a fixed predetermined number of second signals are given to the helper, in order to ventilate the patient with a fixed predetermined number of breaths (ventilation phase), and wherein, in the ventilation phases specified by the device, the oxygen provision is activated by the controller.

2. Device according to Claim 1, **characterized in that** the acoustic signal generator is a loudspeaker.

3. Device according to Claim 1, **characterized in that** the visual signal generator is a colour LED, a display and/or another light-emitting beam source.

4. Device according to Claim 1, **characterized in that** a ventilation valve (1) is connected to the oxygen outlet via a connecting line (8).

5. Device according to Claim 4, **characterized in that** the ventilation valve (1) has an internal volume into which oxygen can be discharged.

6. Device according to one of the preceding claims, **characterized in that** at least one sensor, which detects the ventilation efforts of the helper, is connected to the controller.

7. Device according to one of the preceding claims, **characterized in that** at least one sensor, which detects the cardiac massage by the helper, is connected to the controller.

8. Device according to the two claims above, **characterized in that** when a ventilation effort is detected, the clock generator starts for ventilation and the oxygen valve is open, and/or when a pressure effort is detected, the clock generator starts for cardiac massage and/or the oxygen valve is closed.

9. Device according to one of Claims 1 to 8, **characterized in that**, in the region of the ventilation valve (1), a one-way valve (12) is arranged.

10. Device according to Claim 9, **characterized in that** the one-way valve (12) has a membrane (30).

11. Device according to Claim 9 or 10, **characterized in that** the one-way valve (12) has a spout valve (32).

12. Device according to one of Claims 1 to 11, **characterized in that** the one-way valve (12) in a basic position releases a connection between an inner space of a ventilation mask and an environment.

13. Device according to one of Claims 1 to 12, **characterized in that** the one-way valve (2), in an operating state when pressure is applied by a helper, seals the connecting path between the breathing mask and the environment and releases a passage from a mouthpiece (10) to the interior of the ventilation mask.

14. Method for giving clock pulses for a helper using the device according to Claim 1, the method consisting of at least two phases, wherein the first (cardiac massage) phase and the second (ventilation) phase can have different durations, wherein each phase is identified by frequencies, which can be fixed and predetermined, of acoustic and/or visual signals, and wherein oxygen can be output from the oxygen source via the oxygen valve to the oxygen outlet, **characterized in that** the method is performed by the defibrillator and that in at least one phase the oxygen output is higher than in the other phases, and **in that** a controller of the defibrillator activates oxygen output in ventilation phases.

15. Method according to one of the preceding claims, **characterized in that** the increased oxygen output takes place only in the ventilation phase.

16. Method according to one of the preceding claims, **characterized in that** in the first phase a small oxygen flow escapes from the outlet of the oxygen source.

17. Method according to one of the preceding claims, **characterized in that** a sensor detects ventilation efforts and, after a corresponding detection, the increased oxygen output is introduced.

18. Method according to one of the preceding claims, **characterized in that** the increased oxygen output is output for a fixed period.

19. Method according to one of the preceding claims, **characterized in that** at least one sensor is connected to the controller and detects cardiac massage by the helper.

20. Method according to Claim 19, **characterized in that** when a ventilation effort is detected, the clock generator starts for ventilation and the oxygen valve is open, and/or when a pressure effort is detected, the clock generator starts for cardiac massage and/or the oxygen valve is closed.

## Revendications

1. Dispositif d'attribution d'impulsions de rythme pour un secouriste, qui consiste en un système de commande, qui est raccordé à au moins un générateur de tension, qui peut être relié à au moins une électrode, et est pourvu d'une conduite d'oxygène, reliée à une source d'oxygène, d'une soupape d'oxygène et d'une sortie d'oxygène, ainsi que d'au moins un transmetteur de signaux, qui est conçu pour la transmission de signaux acoustiques et/ou visuels, **caractérisé en ce que** le dispositif est conçu comme une partie d'un défibrillateur, **en ce que** l'électrode est conçue pour la réalisation d'une défibrillation, **en ce que** le générateur de tension est conçu pour la génération d'une tension appropriée pour la réalisation d'une défibrillation et **en ce que** le transmetteur de signaux fournit au secouriste des signaux rythmés pour la fréquence optimale de massage cardiaque par compression thoracique, sachant qu'après un nombre fixe, prédéterminé de premiers signaux, un nombre fixe, prédéterminé de deuxièmes signaux est reçu par le secouriste, afin de ventiler le patient par un nombre d'inspirations fixe (phase de ventilation), prédéterminé, et sachant que, pendant les phases de ventilation prédéterminées par l'appareil, l'administration d'oxygène est libérée par la commande.

2. Dispositif selon la revendication 1, **caractérisé en ce que** le transmetteur de signaux acoustiques est un haut-parleur.

3. Dispositif selon la revendication 1, **caractérisé en ce que** le transmetteur de signaux visuels est une LED de couleur, un affichage et/ou une source de rayonnement, qui émet une lumière quelconque.

4. Dispositif selon la revendication 1, **caractérisé en ce qu'**une valve respiratoire (1) est reliée à la sortie d'oxygène par l'intermédiaire d'une conduite de raccordement (8).

5. Dispositif selon la revendication 4, **caractérisé en ce que** la valve respiratoire (1) dispose d'un volume intérieur, dans lequel l'oxygène peut être déchargé.

6. Dispositif selon l'une des revendications précédentes, **caractérisé en ce qu'**au moins un détecteur, qui détecte les processus de ventilation du secouriste, est relié à l'unité de commande.

7. Dispositif selon l'une des revendications précédentes, **caractérisé en ce qu'**au moins un détecteur, qui détecte les massages cardiaques par compression thoracique, effectués par le secouriste, est relié à l'unité de commande.

8. Dispositif selon les deux revendications précédentes, **caractérisé en ce que**, lors de la détection d'un processus de ventilation, le générateur de rythme est mis en œuvre pour la ventilation et la soupape d'oxygène est ouverte, et/ou que, lors de la détection d'un processus de massage, le générateur de rythme est mis en œuvre pour le massage cardiaque par compression thoracique et/ou la soupape d'oxygène est fermée.

9. Dispositif selon l'une des revendications 1 à 8, **caractérisé en ce qu'**une vanne unidirectionnelle (12) est disposée dans la région de la valve respiratoire (1).

10. Dispositif selon la revendication 9, **caractérisé en ce que** la vanne unidirectionnelle (12) est dotée d'une membrane (30).

11. Dispositif selon la revendication 9 ou 10, **caractérisé en ce que** la vanne unidirectionnelle (12) est dotée d'une soupape à bec (32).

12. Dispositif selon l'une des revendications 1 à 11, **caractérisé en ce que**, dans une position de base, la vanne unidirectionnelle (12) libère une communication entre un espace intérieur d'un masque respiratoire et d'un environnement.

13. Dispositif selon l'une des revendications 1 à 12, **caractérisé en ce que**, dans un état de service, lors de l'exécution d'une compression thoracique par le secouriste, la vanne unidirectionnelle (2) ferme la voie de communication entre le masque respiratoire et l'environnement, et libère un passage entre un embout buccal (10) et l'espace intérieur du masque respiratoire.

14. Procédé d'attribution d'impulsions de rythme pour un secouriste en utilisant le dispositif selon la revendication 1, qui consiste en au moins deux phases, sachant que la première phase, une phase de massage cardiaque par compression thoracique, et la deuxième phase, une phase de ventilation, peuvent avoir de différentes durées de temps, sachant que les phases sont caractérisées chacune par des fréquences de signaux acoustiques et/ou visuels pouvant être prédéterminées de manière fixe, et dans lequel de l'oxygène, en provenance' de la source d'oxygène, peut être délivré à la sortie d'oxygène, par l'intermédiaire' de la soupape d'oxygène, **caractérisé en ce que** le procédé est mis en œuvre par le défibrillateur et **en ce que** l'apport d'oxygène est plus grand dans au moins une phase que dans les autres phases, et **en ce qu'**une commande libère un apport d'oxygène dans les phases de ventilation.

15. Procédé selon l'une des revendications précédentes, **caractérisé en ce que** l'apport plus élevé d'oxygène n'a lieu que dans la phase de ventilation.

16. Procédé selon l'une des revendications précédentes , **caractérisé en ce que**, dans la première phase, un faible flux d'oxygène s'échappe de la sortie de la source d'oxygène.

17. Procédé selon l'une des revendications précédentes, **caractérisé en ce qu'**un détecteur détecte les processus de ventilation, et après une détection adéquate, l'apport d'oxygène plus élevé est initialisé.

18. Procédé selon l'une des revendications précédentes, **caractérisé en ce que** l'apport d'oxygène plus élevé a lieu pendant un laps de temps fixe.

19. Procédé selon l'une des revendications précédentes, **caractérisé en ce qu'**à l'unité de commande est relié au moins un détecteur, qui détecte les massages cardiaques par pression thoracique, exécutés par le secouriste. "

20. Procédé selon la revendication 19, **caractérisé en ce que**, lors de la détection d'un processus de ventilation, le générateur de rythme est mis en œuvre pour la ventilation et la soupape d'oxygène est ouverte et/ou, lors de la détection d'un processus de massage, le générateur de rythme est mis en œuvre pour le massage cardiaque par compression thoracique et/ou la soupape d'oxygène est fermée.
